# EUROPEAN PATENT APPLICATION

(11) **EP 3 539 588 A1**
(43) Date of publication of application: **18.09.2019**
(21) Application number: 17868540.0
(22) Date of filing: 08.11.2017
(51) Int. Cl.: A61M 1/36, G01B 7/16, G01L 7/00, H01L 29/84

(54) **FLUID SYSTEM, MEDICAL SYSTEM, SENSOR, CIRCUIT PIPING MEMBER, ATTACHMENT JIG, AND ATTACHMENT METHOD**

(30) Priority: 10.11.2016 JP 2016220019
(71) Applicant: Dexerials Corporation, Tokyo 141-0032 (JP); Kobe City Hospital Organization, Kobe-shi, Hyogo 6500047 (JP)
(72) Inventor: BABA Yukihisa, Tokyo 141-0032 (JP); MONJU Takuya, Tokyo 141-0032 (JP); YOSHIDA Tetsuya, Kobe-shi Hyogo 650-0047 (JP); HANAOKA Masashi, Kobe-shi Hyogo 650-0047 (JP)
(74) Representative: Murgitroyd & Company
(86) International application number: PCT/JP2017/040298
(87) International publication number: WO 2018/088438

(57) **Abstract**

A fluid system 10 according to the present disclosure includes an EAP sensor 13 disposed on an outer surface of a circuit conduit 11. The EAP sensor 13 includes a polymer element 131 including electrode layers 133A, 133B provided at respective surfaces of an ion-conductive polymer layer 132. The EAP sensor 13 is configured to output a signal corresponding to a deformation of the circuit conduit 11.

## Description

### CROSS REFERENCE TO RELATED APPLICATIONS

The present application claims a priority on Japanese Patent Application No. 2016-220019, filed on November 10, 2016, the entire contents of which are incorporated herein by reference.

### TECHNICAL FIELD

The present disclosure relates to a fluid system that allows a passage of a fluid through a circuit having a circuit conduit, a medical system, a sensor, a circuit conduit member, an attachment jig, and an attachment method.

### BACKGROUND

An extracorporeal membrane oxygenation (ECMO) is an extracorporeal circulatory life support system, in which blood (venous blood) is drawn from a living human body, and the drawn blood is sent to an artificial lung by a pump. The artificial lung then provides oxygen to and removes carbon dioxide from the blood, and the resultant blood is returned to a vein or artery of the patient. ECMOs are employed for some of possibly reversible life-threatening medical conditions, e.g., severe respiratory or circulatory failures, which had been considered fatal under conventional medical procedures, such as managements with mechanical ventilators and vasopressor agents.

In recent years, in the medical or other industries, applications of a polymer element have been researched, which includes a polymer layer (ion-conductive polymer layer) and a pair of electrode layers formed at respective surfaces of the polymer layer. Such a polymer element deforms in response to an application of an electrical field to the pair of electrode layers, as well as outputting an electric signal corresponding to a deformation caused by an external force.

For example, Patent Literature 1 (PTL1) discloses a technique wherein a polymer element is disposed on the circumferential surface of a tubular main body, and an application of an electrical field to electrode layers of the polymer element causes a deformation of the tubular main body.

Furthermore, Patent Literature 2 (PTL2) discloses a technique wherein a polymer element is disposed inside a fluid conduit that allows passage of a fluid, and an application of an electrical field to electrode layers causes a deformation of the polymer element, and the deformation is used to control the characteristics of the fluid flowing through the fluid conduit.

### CITATION LIST

### Patent Literature

PTL 1: JP 2010-252545-A
PTL 2: JP 2005-527178-A

### SUMMARY

### (Technical Problem)

In ECMOs described above, excessive negative or positive pressures inside circuit conduits where blood circulates are undesirable. A trifurcating circuit, hence, may be provided at a circuit conduit, and a pressure sensor for detecting the pressure inside the circuit conduit may be connected to the trifurcating circuit. Provision of such a trifurcating circuit, however, may increase the risk of thrombi and may also compromise workability.

Circuit conduits used in an ECMO are generally configured from flexible tubes made of vinyl chloride or other materials. If an excessive negative or positive pressure is created inside such a circuit conduit, the circuit conduit deforms, i.e., the diameter thereof changes (a contraction or expansion occurs). If such a deformation of the circuit conduit is detected, an excessive negative or positive pressure inside a circuit conduit can be detected without requiring any pressure sensors. No particular methods of detecting deformations of a circuit conduit, however, have been studied extensively.

In the above, descriptions have been made with reference to the example of an extracorporeal circulatory blood circulation system (ECMO) for circulating blood in a circulatory circuit outside a living body. Detections of excessive negative or positive pressures, however, are in need in a wide variety of fluid systems that allow a passage of a fluid in a circuit having a circuit conduit.

An object of the present disclosure is to solve the above-identified issues, and is to provide a fluid system, a medical system, a sensor, and a circuit conduit member that can detect deformations of a circuit conduit caused by fluctuations in the pressure inside the circuit conduit.

### (Solution to Problem)

In order to solve the above-identified issues, a fluid system in accordance with the present disclosure is a fluid system that allows a passage of a fluid through a circuit having a circuit conduit, the fluid system including a sensor disposed on an outer surface of the circuit conduit, the sensor including a polymer element including electrode layers provided at respective surfaces of an ion-conductive polymer layer, wherein the sensor is configured to output a signal corresponding to a deformation of the circuit conduit.

Furthermore, in the fluid system in accordance with the present disclosure, the sensor is preferably secured to the outer surface of the circuit conduit by a protective film.

Furthermore, in the fluid system in accordance with the present disclosure, the ion-conductive polymer layer is preferably a polymer layer made of a fluororesin having a polar group.

Furthermore, in the fluid system in accordance with the present disclosure, preferably, the fluid system allows the passage of the fluid in the circuit using a pump, the circuit conduit includes a first line that allows a transportation of the fluid toward the pump and a second line that allows a transportation of the fluid away from the pump, and the sensor is disposed on an outer surface of at least one of the first line and the second line.

Furthermore, in the fluid system in accordance with the present disclosure, preferably, the polymer element has an approximate rectangular shape in plan view, in a case where the sensor is disposed on an outer surface of the first line, the sensor is disposed such that long sides of the polymer element extend along a radial direction of the first line, and in a case where the sensor is disposed on an outer surface of the second line, the sensor is disposed such that long sides of the polymer element extend along a longitudinal direction of the second line.

Furthermore, in the fluid system in accordance with the present disclosure, the fluid is preferably a liquid, such as blood. In place of blood, the fluid may be liquids related to raw materials used for organic syntheses, drugs, or life-care products.

Furthermore, in the fluid system in accordance with the present disclosure, preferably, a voltage corresponding to a deformation of the circuit conduit is induced in the sensor, and an electromotive force difference has a proportional relationship with an absolute value of a pressure inside the circuit conduit, the electromotive force difference being a difference between the induced voltage in the sensor corresponding to the deformation of the circuit conduit caused by a pressure inside the circuit conduit, and an induced voltage in the sensor in a predetermined condition.

Furthermore, in order to solve the above-identified issues, a medical system in accordance with the present disclosure includes the aforementioned fluid system, and a processor configured to carry out a predetermined process according to the signal output from the sensor.

Furthermore, in order to solve the above-identified issues, a sensor in accordance with the present disclosure is used in any one of the aforementioned fluid systems, and the sensor is configured to output the signal corresponding to the deformation of the circuit conduit.

Furthermore, in order to solve the above-identified issues, a circuit conduit member in accordance with the present disclosure includes the aforementioned sensor disposed on an outer surface thereof.

Furthermore, in order to solve the above-identified issues, an attachment jig in accordance with the present disclosure is a jig for attaching the aforementioned sensor to the circuit conduit, the jig including a clamping section having an arc shape in cross-sectional view; and a pressing section accommodated within the arc of the clamping section, wherein the sensor is to be placed on a surface of the pressing section opposite to a surface facing the clamping section, and an accommodation of the circuit conduit within the arc of the clamping section causes the sensor to be pressed by the pressing section against an outer surface of the circuit conduit.

Furthermore, in order to solve the above-identified issues, a method of attaching in accordance with the present disclosure is a method of attaching the sensor to the circuit conduit using the aforementioned attachment jig, the method including placing the sensor on the surface of the pressing section opposite to the surface facing the clamping section; accommodating the circuit conduit within the arc of the clamping section, thereby pressing the sensor by the pressing section against the outer surface of the circuit conduit; and securing the clamping section and the circuit conduit to each other with a securing member.

### (Advantageous Effect)

According to the fluid system, the medical system, the sensor, the circuit conduit member, the attachment jig, and the attachment method in accordance with the present disclosure, a deformation of a circuit conduit caused by fluctuations in the pressure in a circuit conduit can be detected.

### BRIEF DESCRIPTION OF THE DRAWINGS

In the accompanying drawings:
FIG. 1 is a diagram depicting an exemplary configuration of a fluid system in accordance with an embodiment of the present disclosure;
FIG. 2 is a diagram depicting an exemplary schematic configuration of a polymer element provided in the EAP sensor depicted in FIG. 1;
FIG. 3 is a diagram depicting an exemplary configuration of a medical system provided with the fluid system depicted in FIG. 1;
FIG. 4 is a cross-sectional view depicting an exemplary configuration of an attachment jig in accordance with the present disclosure;
FIG. 5 is a perspective view depicting a clamping section depicted in FIG. 4;
FIG. 6A is a diagram illustrating a method of attaching an EAP sensor to a circuit conduit with the attachment jig depicted in FIG. 5 (Part 1);
FIG. 6B is a diagram illustrating a method of attaching the EAP sensor to the circuit conduit with the attachment jig depicted in FIG. 5 (Part 2);
FIG. 7 is a diagram depicting an EAP sensor of Example 1;
FIG. 8 is a diagram depicting an EAP sensor of Example 2;
FIG. 9 is a diagram depicting an EAP sensor of Example 3;
FIG. 10 is a diagram depicting an EAP sensor of Example 4;
FIG. 11 is a diagram depicting an EAP sensor of Example 5;
FIG. 12 is a diagram depicting an EAP sensor of Example 6;
FIG. 13 is a diagram depicting an EAP sensor of Example 7;
FIG. 14 is a diagram depicting an exemplary adherence of an EAP sensor;
FIG. 15A is a diagram depicting exemplary waveforms of induced voltages in the EAP sensor of Example 1 under negative pressure conditions;
FIG. 15B is a diagram depicting exemplary waveforms of induced voltages in the EAP sensor of Example 7 under negative pressure conditions;
FIG. 16A is a diagram depicting exemplary waveforms of induced voltages in the EAP sensor of Example 1 under positive pressure conditions;
FIG. 16B is a diagram depicting exemplary waveforms of induced voltages in the EAP sensor of Example 7 under positive pressure conditions;
FIG. 17A is a graph indicating the relationship between positive pressures in a circuit conduit and electromotive force differences in the EAP sensor; and
FIG. 17B is a graph indicating the relationship between negative pressures in the circuit conduit and electromotive force differences in the EAP sensor.

### DETAILED DESCRIPTION

Embodiments of the present disclosure will be described below.

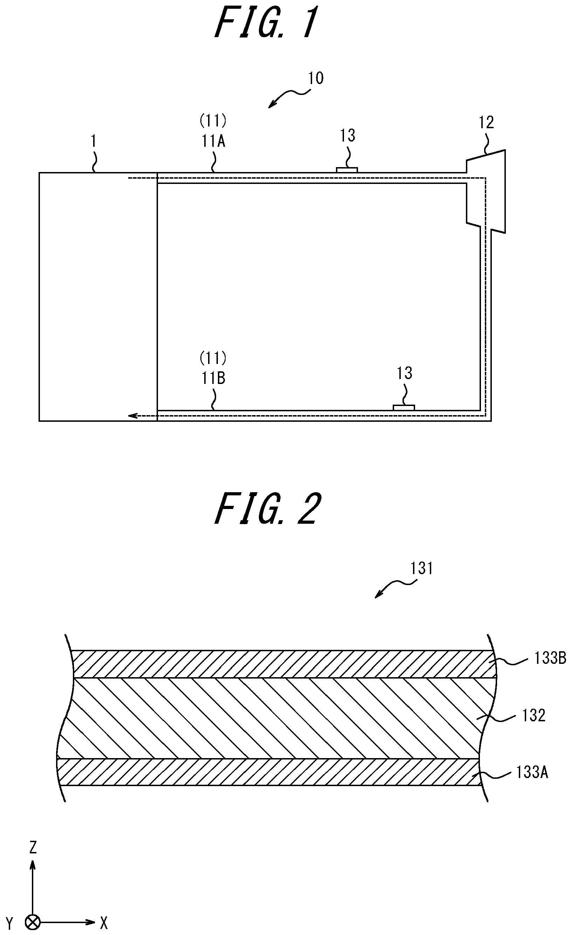

FIG. 1 is a diagram depicting an exemplary configuration of a fluid system 10 in accordance with an embodiment of the present disclosure. In FIG. 1, descriptions will be made with reference to an example of an application of the fluid system 10 to a liquid circulation system where liquid is drawn from a liquid source 1 and is then returned to the liquid source 1. This, however, is not limiting, and the present disclosure is also applicable to liquid feeding systems where liquid is drawn from the liquid source 1 and is then fed to a certain destination. While descriptions of FIG. 1 will be made with reference to the exemplary fluid system 10 that allows a passage of liquid, applications to a system that allows passages of non-liquid fluids, e.g., gases, are also possible.

The fluid system 10 depicted in FIG. 1 includes a liquid drawing line 11A (first line), a liquid feeding line 11B (second line), a pump 12, and an electro-active polymer (EAP) sensor 13. The liquid drawing line 11A and the liquid feeding line 11B are collectively referred to as the "circuit conduit 11."

The circuit conduit 11 is a tubular member that allows a transportation of liquid. The materials used to form the circuit conduit 11 include thermoplastic elastomers, including those based on styrenes, polyolefins, polyurethanes, polyesters, polyamides, polybutadienes, trans-polyisoprenes, fluororubbers, and chlorinated polyethylenes; and one of these or combinations of two or more of these (e.g., polymer alloys, polymer blends, and stacks) may be used.

The liquid drawing line 11A allows a transportation of liquid drawn from the liquid source 1, toward the pump 12. The liquid feeding line 11B allows a transportation of liquid sent by the pump 12, toward the liquid source 1. In this manner, the circuit conduit 11 is configured such that liquid drawn from the liquid source 1 is circulated through the circuit conduit 11 and is then returned to the liquid source 1.

The pump 12 is connected to the liquid drawing line 11A and the liquid feeding line 11B, and sends out liquid flowing into the pump 12 through the liquid drawing line 11A, toward the liquid source 1 through the liquid feeding line 11B.

In this manner, the fluid system 10 allows circulations of fluid (liquid) where the fluid (liquid) is drawn from the liquid source 1 through a circuit having the circuit conduit 11 (the liquid drawing line 11A and the liquid feeding line 11B) and the pump 12, and is returned to the liquid source 1.

The EAP sensor 13 is a sensor that is disposed (secured) on an outer surface of the circuit conduit 11 (at least one of the liquid drawing line 11A and the liquid feeding line 11B), and outputs an electric signal corresponding to a deformation of the circuit conduit 11. The EAP sensor 13 is secured (adhered) to the circuit conduit 11 by an adhesive protective film, for example.

The EAP sensor 13 includes a polymer element including a polymer layer (ion-conductive polymer layer) and electrode layers. The EAP sensor 13 deforms in conjunction with a deformation of the circuit conduit 11 having the EAP sensor 13 disposed threreon, and outputs an electric signal induced in response to the deformation from terminals connected to the electrode layers.

In place of adhering the EAP sensor 13 to the circuit conduit 11, a tubular circuit conduit member having an EAP sensor 13 integrated thereto may be provided as a circuit conduit 11, and may be connected to the liquid source 1 and the pump 12.

FIG. 2 is a diagram depicting an exemplary schematic configuration (exemplary configuration on the Z-X plane) of the polymer element 131 provided in the EAP sensor 13.

The polymer element 131 depicted in FIG. 2 includes a polymer layer 132 (ion-conductive polymer layer) and electrode layers 133A and 133B.

The polymer layer 132 is made from any of ion-conductive polymers, including fluororesins having one or more polar groups, e.g., sulfonic acid groups or carboxylic acid groups, such as Nafion® (Nafion is a registered trademark in Japan, other countries, or both) manufactured by Du Pont Corporation. Note that the materials used to form the polymer layer 132 are not limited to these, and the polymer layer 132 may be made from any ion-conductive polymeric compounds having ionic materials impregnated therein. The term "ionic materials" collectively refers to any ions that can conduct through the polymer layer 132, and may be organic or inorganic materials. For example, non-limiting examples of ionic materials include hydrogen ions or elemental metal ions; mixtures of such cations and/or anions and polar solvents; and materials including intrinsically liquefied cations and/or anions, e.g., imidazolium salts.

The electrode layers 133A and 133B are provided at the respective surfaces of the polymer layer 132, so as to sandwich the polymer layer 132 along the Z direction. Each of the electrode layers 133A and 133B is provided by adding carbon blacks to the polymer material of the polymer layer 132.

When the polymer element 131 deforms (bends) under an external force in the positive direction of the Z axis (the direction toward the electrode layer 133B) in FIG. 2, for example, the electrode layer 133B side compresses and the electrode layer 133A side expands, in the polymer layer 132. This causes cations contained in the polymer layer 132 to migrate toward the electrode layer 133A side where the internal pressure is lower. This results in a higher cation level on the electrode layer 133A side, and a lower cation level on the electrode layer 133B side. This gradient of the ion level induces a potential difference between the electrode layers 133A and 133B, which is output in the form of an electric signal from output terminals (not illustrated in FIG. 2) connected to the electrode layers 133A and 133B.

As set forth above, the EAP sensor 13 is secured to the outer surface of the circuit conduit 11. Thus, when the polymer element 131 deforms in conjunction with a deformation of the circuit conduit 11 having the EAP sensor 13 is disposed thereon, the deformation of the polymer element 131 induces a potential difference between the electrode layers 133A and 133B. The EAP sensor 13 outputs this potential difference in the form of an electric signal from the output terminals.

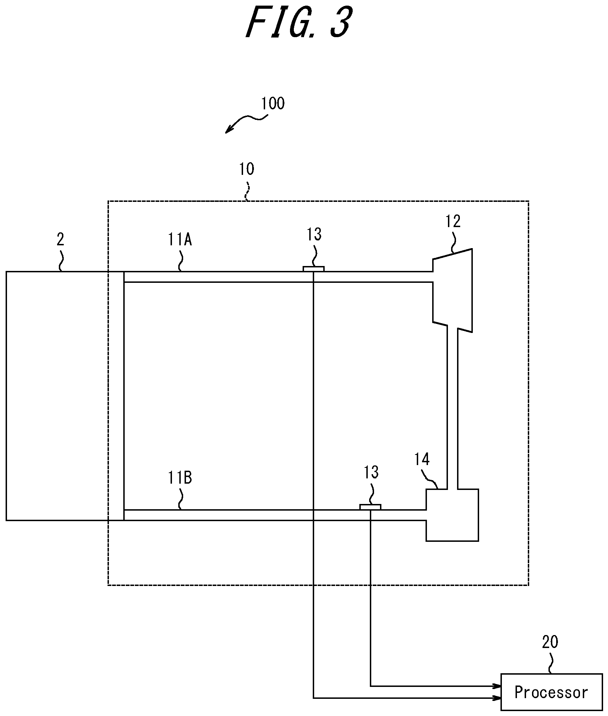

FIG. 3 is a diagram depicting an exemplary configuration of a medical system 100 provided with the fluid system 10 according to this embodiment. The medical system 100, for example, is a system used in intensive care units in hospitals.

The medical system 100 depicted in FIG. 3 includes a fluid system 10 and a processor 20. Note that like reference symbols in FIG. 3 denote elements similar to the ones in the FIG. 1, and the descriptions on such elements are omitted.

FIG. 3 illustrates an exemplary application of the fluid system 10 to an extracorporeal membrane oxygenation (ECMO). In this case, the fluid system 10 further includes an artificial lung 14. The fluid system 10 sends blood (venous blood) drawn from a living human body 2 (patient) to the artificial lung 14 using a pump 12. The artificial lung 14 provides oxygen to and removes carbon dioxide from the blood sent from the pump 12, and the blood that underwent the oxyganation and the removal of carbon dioxide is returned to a vein or artery in the living human body 2.

In response to deformation of the circuit conduit 11 (the liquid drawing line 11A and/or the liquid feeding line 11B), the EAP sensor 13 outputs an electric signal corresponding to the deformation of the circuit conduit 11, to the processor 20.

The processor 20 carries out a predetermined process in accordance with the electric signal output from the EAP sensor 13. Specific examples of the processor 20 are alarm devices that issue alarms in response to an excessive negative or positive pressure inside the circuit conduit 11. The processor 20 may also functions as an electrocardiograph that graphically displays and records electrical activities of the heart of the patient. In this case, the processor 20 displays and records waveforms indicating the electrical activities of the heart of the patient, as well as waveforms of electric signals output from the EAP sensor 13.

Although the example has been described wherein the fluid flowing through the fluid system 10 is liquid blood in FIG. 3, this is not limiting. The fluid flowing through the fluid system 10 may be liquids related to raw materials used for organic syntheses, drug-related liquids, liquids related to life-care products, e.g., cosmetics, or any other liquids.

Alternatively, the EAP sensor 13 according to this embodiment may be attached to food-related liquid feeding tubes that need to satisfy certain hygiene requirements, such as tubes for beer servers, for example. An EAP sensor 13 may be attached to the outer surface of a tube for a beer server such that the EAP sensor 13 can detect deformations of the tube. This enables the EAP sensor 13 to detect the pressure inside the tube based on the deformation of the tube, and appropriate timing to exchange beer barrels can be determined in hygienic manner without requiring installation of in-tube sensors. The EAP sensor 13 according to this embodiment may also be attached to brake hoses of automobiles for allowing a passage of brake oil. This enables detections of abnormal pressures inside brake hoses without requiring any additional tubing and pressure sensors inside the brake hoses.

As set forth above, in this embodiment, the fluid system 10 includes the EAP sensor 13 disposed on the outer surface of the circuit conduit 11, the EAP sensor 13 includes the polymer element 131 including the electrode layers 133A and 133B provided at the respective surfaces of the polymer layer 132, and the EAP sensor 13 is configured to output a signal corresponding to a deformation of the circuit conduit 11.

When the polymer element 131 having the electrode layers 133A and 133B provided at the respective surfaces of the polymer layer 132 deforms, a potential difference arises between the electrode layers 133A and 133B. The disposition of the EAP sensor 13 having that polymer element 131 on the outer surface of the circuit conduit 11 permits the polymer element 131 to deform in conjunction with a deformation of the circuit conduit 11, when the circuit conduit 11 experiences a pressure inside the circuit conduit 11. The deformation of the circuit conduit 11 induces a potential difference between the electrode layers 133A and 133B, which is provided as an electric signal. As a result, the deformation of the circuit conduit 11 induced by the pressure inside the circuit conduit 11 can be detected.

Further, in the fluid system 10 according to this embodiment, the EAP sensor 13 is disposed on the outer surface of the circuit conduit 11. A deformation of the circuit conduit 11 is detected without creating any stagnation of flow in the circuit conduit 11 or introduction of foreign objects inside the circuit conduit 11. Therefore, when the fluid system 10 is applied to an extracorporeal circulatory blood circulation system, the risks of thrombi are reduced, for example.

In a non-limiting method of attaching an EAP sensor 13 to the circuit conduit 11, the above-described EAP sensor 13 is secured to the circuit conduit by placing it around the circuit conduit while applying a tension with a flexible urethane sheet. Hereinafter, an attachment jig and an attachment method using that attachment jig according to this embodiment will be described.

Initially, an attachment jig will be described.

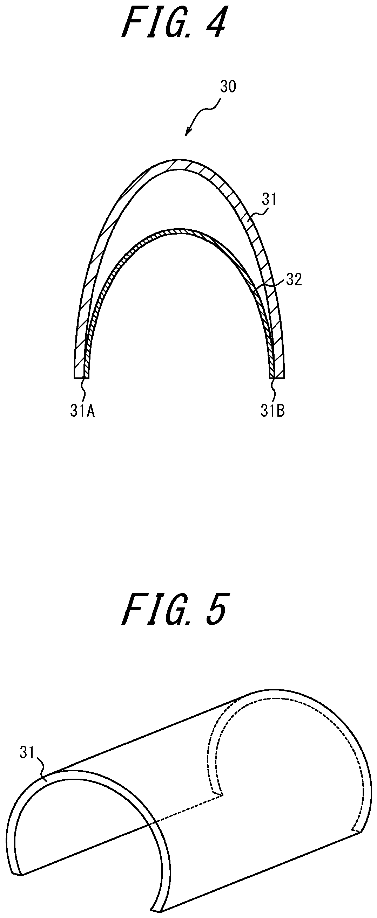

FIG. 4 is a cross-sectional view depicting the configuration of an attachment jig 30.

The attachment jig 30 depicted in FIG. 4 includes a clamping section 31 and a pressing section 32. FIG. 5 is a perspective view depicting the clamping section 31.

As depicted in FIG. 5, the clamping section 31 is a tubular member having an arc shape in cross-sectional view, and a part of a tube has been cut out in parallel to the longitudinal direction of the tubular member. The clamping section 31 is preferably made from a resin, such as polypropylene (PP), acrylonitrile-butadiene-styrene (ABS), and polycarbonate (PC), for example.

In the cross-sectional diagram in FIG. 4 (in the cross-sectional view), respective ends of the pressing section 32 are secured to one end 31A and the other end 31B of the arc-shaped clamping section 31. When the pressing section 32 is flattened, the width of the pressing section 32 is greater than the gap between the one end 31A and the other end 31B of the clamping section 31. Thus, the pressing section 32 is secured in the loosened state between the one end 31A and the other end 31B of the clamping section 31, so as to be accommodated within the arc of the clamping section 31. The pressing section 32 is preferably made from an elastic film, such as a urethane film, a rubber sheet, and an olefin film, and extends along the longitudinal direction of the clamping section 31.

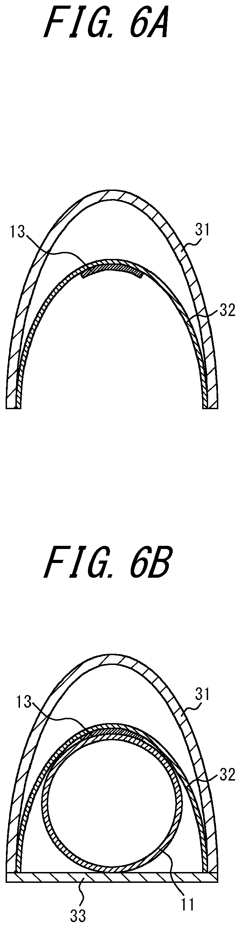

Next, referring to FIGS. 6A and 6B, a method of attaching the EAP sensor 13 to the circuit conduit 11 using the attachment jig 30 will be described.

First, as depicted in FIG. 6A, the EAP sensor 13 is placed (adhered) on the surface of the pressing section 32 which is opposite to the surface facing the clamping section 31. Next, as depicted in FIG. 6B, the circuit conduit 11 is accommodated within the arc-shaped clamping section 31. The accommodation of the circuit conduit 11 within the clamping section 31 causes the EAP sensor 13 adhered to the pressing section 32 to be pressed against the outer surface of the circuit conduit 11, as depicted in FIG. 6B. The clamping section 31 and the circuit conduit 11 are then secured to each other with a securing member 33, from the opening side of the arc-shaped clamping section 31. In this manner, the EAP sensor 13 is secured to the circuit conduit 11 while the EAP sensor 13 is pressed against the circuit conduit 11.

Note that the securing member 33 is not limed to a flat plate member as the one depicted in FIG. 6B, and any jigs suitable for fixations to tubes, e.g., resin snappers, resin tying bands, and hose fixation fittings, may be used.

### EXAMPLES

Next, the present disclosure will be described more concretely with reference to the following non-limiting examples.

### (Example 1)

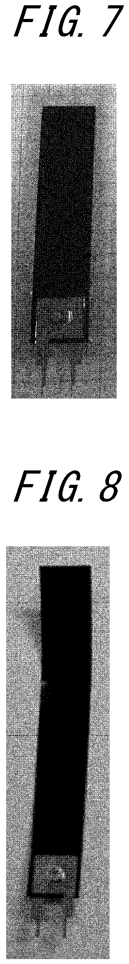

In this example, as depicted in FIG. 7, an EAP sensor was fabricated which included a rectangular polymer element with a width of 1 cm and a length of 5 cm, and output terminals extending along the long side direction from an end of the polymer element.

The polymer element was fabricated as follows:

Initially, as described in Paragraph 0037 in JP 2010-252545-A, a paint containing powders of a conductive material and conductive polymers dispersed in a dispersion medium was applied on the two surfaces of an ion-conductive polymer film. The dispersion medium was then allowed to evaporate, to form electrode layers on the two surfaces of the ion-conductive polymer film, and cationic materials were then impregnated into the ion-conductive polymer films. The ion-conductive polymer film and the electrode layer were cut into the predetermined size (1 cm × 5 cm in Example 1) to fabricate the polymer element. Output terminals were attached to the polymer element to fabricate an EAP sensor. These processes were also used to fabricate EAP sensors in the following Examples.

### (Example 2)

In this example, as depicted in FIG. 8, an EAP sensor was fabricated which included a rectangular polymer element with a size of 1 cm × 7 cm, and output terminals extending along the long side direction from an end of the polymer element.

### (Example 3)

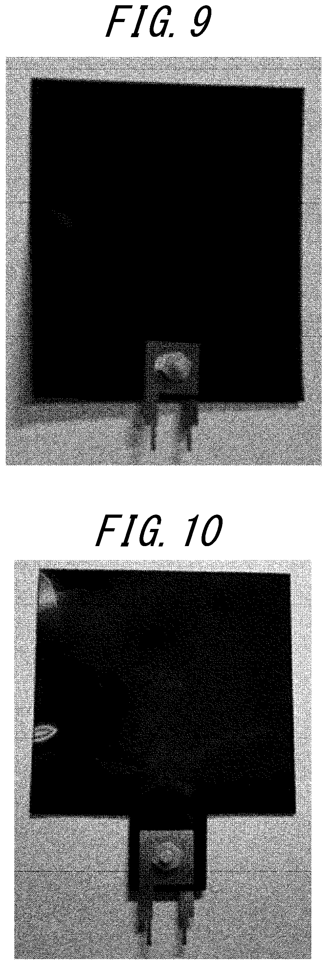

In this example, as depicted in FIG. 9, an EAP sensor was fabricated which included a rectangular polymer element with a size of 4 cm × 5 cm, and output terminals extending in the long side direction, from an approximate center of a short side of the polymer element.

### (Example 4)

In this example, as depicted in FIG. 10, an EAP sensor was fabricated which included a square polymer element with a size of 4 cm × 4 cm having a protrusion protruding from an approximate center of one side, and output terminals provided at the protrusion.

### (Example 5)

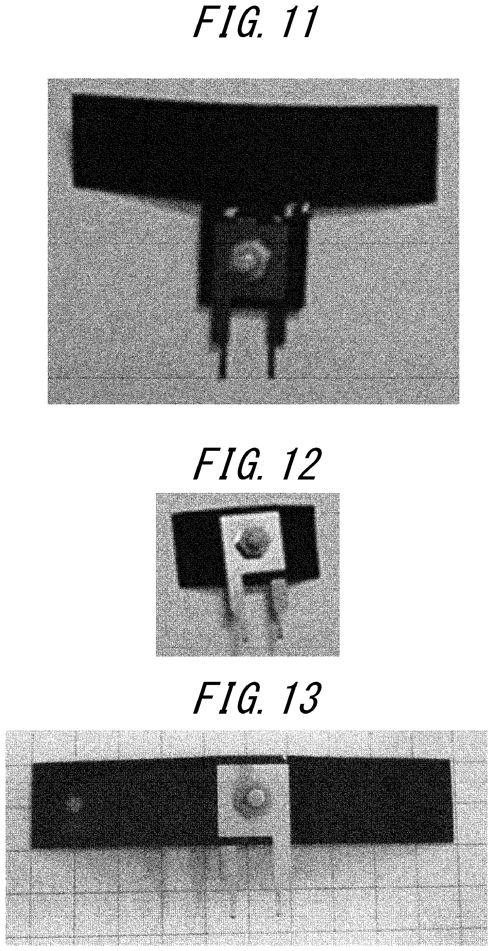

In this example, as depicted in FIG. 11, an EAP sensor was fabricated which included a rectangular polymer element with a size of 4 cm × 1 cm having a protrusion protruding from an approximate center of one long side, and output terminals provided at the protrusion.

### (Example 6)

In this example, as depicted in FIG. 12, an EAP sensor was fabricated which included a rectangular polymer element with a size of 2 cm × 1 cm, and output terminals extending along the short side direction, from an approximate center in the long side direction.

### (Example 7)

In this example, as depicted in FIG. 13, an EAP sensor was fabricated which included a rectangular polymer element with a size of 5 cm × 1 cm, and output terminals extending along the short side direction, from an approximate center in the long side direction.

### (Measurements of Voltages induced in EAP Sensors)

Next, induced voltages were measured in the EAP sensors of Examples 1-7. Firstly, how to measure induced voltages in the EAP sensors of Examples 1-7 will be described.

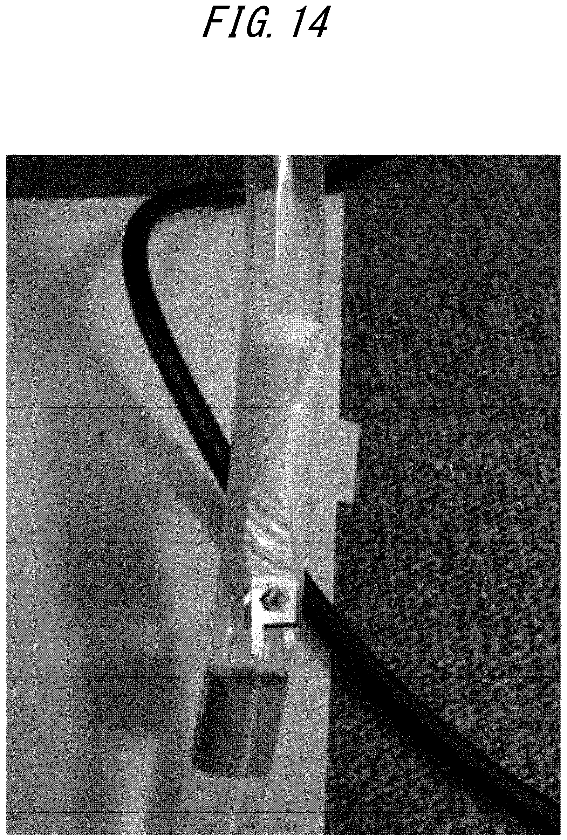

As a circuit conduit 11 (liquid drawing line 11A and liquid feeding lines 11B), transparent tubes made from vinyl chloride (having an outer diameter 14.2 mm and an inner diameter of 9.5 mm) were used. The EAP sensors of Examples 1-7 were secured to the transparent tubes, by placing them around the transparent tubes while applying tensions with a flexible urethane sheet (see FIG. 14).

One end of a transparent tube as a liquid drawing line 11A having an EAP sensor secured thereto was coupled to a casing containing tap water, and the other end was connected to a centrifugal pump. One end of a transparent tube as a liquid feeding line 11B was coupled to the casing containing tap water, and the other end was connected to the centrifugal pump. The tap water in the casing was maintained at about 35°C by a heater. The tap water was fed and circulated by the centrifugal pump manufactured by Rouchon Industries Inc. under the product name of "MCP655-B." A negative pressure condition was created inside a transparent tube as the liquid drawing line 11A by completely closing a water supply side inlet of the transparent tube as the liquid drawing line 11A with a thumb or finger while the tap water was being fed, and an induced voltage in the EAP sensor was measured in this condition.

The induced voltages in the EAP sensor of Examples 1-7 are listed in Table 1.

**Table 1**

| | Induced Voltages |
|---|---|
| Example 1 | 0.035 mV |
| Example 2 | 0.026 mV |
| Example 3 | 0.018 mV |
| Example 4 | 0.014 mV |
| Example 5 | 0.032 mV |
| Example 6 | 0.002 mV |
| Example 7 | 0.036 mV |

As indicated in Table 1, in all of the EAP sensors of Examples 1-7, the negative pressures inside the transparent tubes as the liquid drawing lines 11A induced voltages, which enabled detections of deformations of the transparent tubes as the liquid drawing lines 11A.

Generally, an induced voltage tends to lower with an increase in the size of an EAP sensor. The reason is considered that, in a larger EAP sensor, a portion of a polymer element may not deform when a circuit conduit deforms, resulting in a decline in the front-back volume ratio (i.e., the ratio of the volume of the contracting side to the volume of an expanding side upon a deformation). Therefore, the size and the shape of an EAP sensor are needed to be determined such that the front-back volume ratio varies significantly in response to a deformation of the circuit conduit.

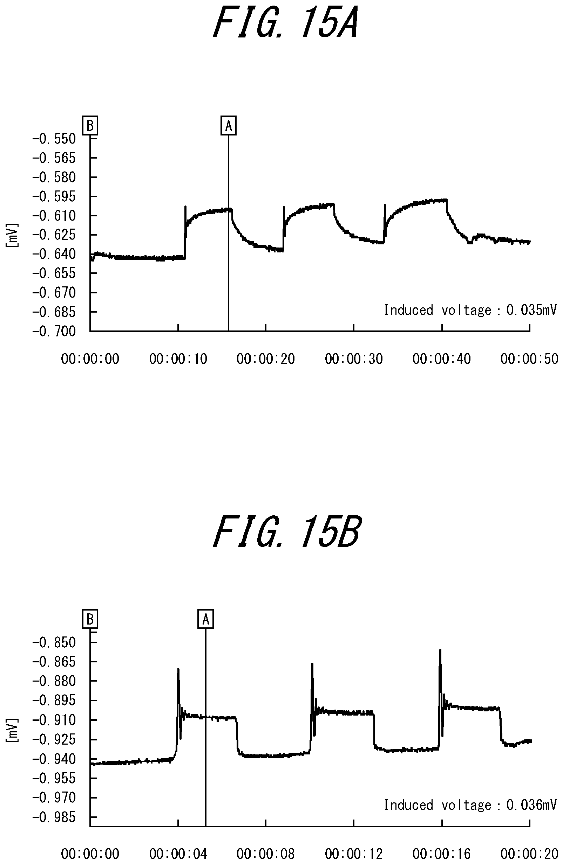

In the measurements under negative pressure conditions, higher voltages were induced in the EAP sensors of Examples 1 and 7. FIGS. 15A and 15B are diagrams indicating waveforms of induced voltages in the EAP sensors of Examples 1 and 7 under the negative pressure conditions, respectively.

Note that the EAP sensor of Example 1 has the polymer element with the same size as that in the polymer element in the EAP sensor of Example 7, although one is vertically long and the other is horizontally long. The direction to adhere the EAP sensor of Example 1 to the transparent tube was different from that in the EAP sensor of Example 7. More specifically, the EAP sensor of Example 1 was disposed such that the long sides of the polymer element extended along the longitudinal direction of the transparent tube. In contrast, the EAP sensor of Example 7 was disposed such that the long sides of the polymer element extended along the radial direction of the transparent tube.

As indicated in Table 1, there are no significant differences between the induced voltages in the EAP sensors of Examples 1 and 7 under the negative pressure conditions. The waveforms of the induced voltages in the EAP sensors indicated in FIGS. 15A and 15B, however, indicate that the EAP sensor of Example 7 exhibited excellent (rapid) increases in the induced voltages. This suggests that the EAP sensor of Example 7 has a better followability to deformations of a transparent tube.

As set forth above, the EAP sensors of Examples 1 and 7 are different in terms of adherence directions of the polymer elements, although the sizes of the polymer elements are the same. Accordingly, for highly sensitive detections of deformations of a circuit conduit caused by negative pressures (deformations of a liquid drawing line that may experience negative pressures), an EAP sensor having a rectangular polymer element is preferably disposed such that the long sides of the polymer element extend along the radial direction of the circuit conduit, in the manner similar to the adherence of the EAP sensor of Example 7.

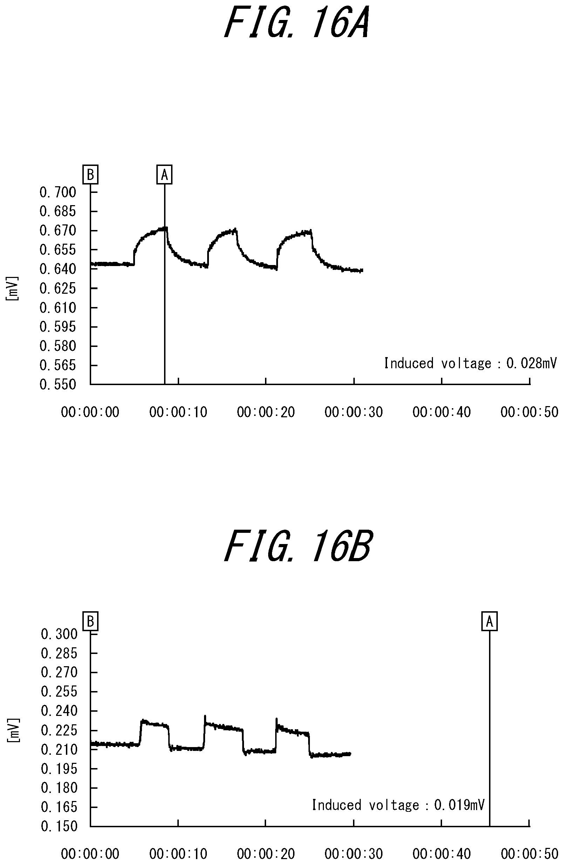

Next, induced voltages in the EAP sensors of Examples 1 and 7 under positive pressure conditions were measured. FIGS. 16A and 16B are diagrams indicating waveforms of induced voltages in the EAP sensors of Examples 1 and 7 under the positive pressure conditions, respectively.

Upon measurements of induced voltage under the positive pressure conditions, a positive pressure condition was created inside a circuit conduit as the liquid feeding line 11B by pinching the transparent tube as the liquid feeding line 11B with forceps. The directions to adhere the EAP sensors of Examples 1 and 7 were the same as those upon the measurements of the induced voltages under the negative pressure conditions.

As depicted in FIGS. 16A and 16B, under the positive pressure conditions, the EAP sensor of Example 7 exhibited more rapid rises in the induced voltages as compared to the EAP sensor of Example 1. The induced voltage in the EAP sensor of Example 7, however, was 0.019 mV whereas the induced voltage in the EAP sensor of Example 1 was 0.028 mV. Higher induced voltages are preferable for detections of deformations of a circuit conduit. Accordingly, for detections of deformations of the circuit conduit caused by positive pressures (deformations of the liquid feeding line that may experience positive pressures), an EAP sensor having a rectangular polymer element is preferably disposed such that the long sides of the polymer element extend along the longitudinal direction of the circuit conduit.

(Study of Relationship between Pressures inside Circuit Conduit and Induced Voltages (Electromotive Forces))

Next, the relationship between pressures inside a circuit conduit and induced voltages (electromotive forces) in an EAP sensor was studied. Initially, how the relationship between pressures in a circuit conduit and induced voltages in an EAP sensor was studied will be described.

The relationship between pressures inside a circuit conduit and induced voltages in an EAP sensor was studied using an ECMO system (percutaneous cardio-pulmonary support system CAPIOX EBS and Custom Pack manufactured by Terumo Corporation). To the piping of this ECMO system, a tube as a liquid drawing line 11A (hereinafter referred to as "liquid drawing tube") and a tube as a liquid feeding line 11B (hereinafter referred to as "liquid feeding tube") were connected. As the liquid drawing tube, CAPIOX catheter kit (18-Fr) manufactured by Terumo Corporation was used. As the liquid feeding tube, CAPIOX catheter kit (13.5-Fr) manufactured by Terumo Corporation was used. Physiological saline was suctioned from a tank containing physiological saline and was fed, thereby the physiological saline was circulated through the ECMO system. A heating and cooling water tank (MERA Small heating and cooling unit manufactured by SENKO MEDICAL INSTRUMENT Mfg. CO., LTD. under the product name of "HHC-51") was connected to an artificial lung in the ECMO system, thereby the physiological saline was maintained at about 37°C (room temperature was 25°C).

Respective EAP sensors of Example 1 were attached to the liquid drawing and liquid feeding tubes. In addition, monitor kit for measuring liquid drawing pressures (negative pressures) was attached to the liquid drawing tube. Monitor kit for measuring liquid feeding pressures (positive pressures) was attached to the liquid feeding tube. As these monitor kits, blood pressure monitor sets manufactured by Argon Medical Devices Japan, Inc., were used.

In the measurement system described above, after the liquid drawing tube or liquid feeding tube was occluded externally with forceps while physiological saline was being circulated, thereby creating a positive or negative pressure condition inside the circuit conduit, an induced voltage in the EAP sensor was measured.

Specifically, measurements of liquid feeding pressures (positive pressures), .i.e., measurements of positive pressures, were carried out using the monitor kit attached to the liquid feeding tube, simultaneously with reading of signals (induced voltages) from the EAP sensor attached to the liquid feeding tube. More specifically, after the pressure inside the liquid feeding tube was adjusted to +123 mmHg as an initial pressure, the outlet (casing side end) of the liquid feeding tube was pinched with forceps to change the pressure inside the liquid feeding tube to +200, +300, +400, and +500 mmHg. Then, the electromotive force differences, i.e., the differences between induced voltages (electromotive forces) in the EAP sensor and the initial voltage (induced voltage under the initial pressure, i.e., predetermined condition), were determined.

Furthermore, measurements of liquid drawing pressures (negative pressures), .i.e., measurements of negative pressures, were carried out using the monitor kit attached to the liquid drawing tube, simultaneously with reading of signals (induced voltages) from the EAP sensor attached to the liquid drawing tube. Specifically, after the pressure inside the liquid drawing tube was adjusted to -64 mmHg as an initial pressure, the inlet (casing side end) of the liquid drawing tube was pinched with forceps to change the pressure inside the liquid drawing tube to -150, -200, and -250 mmHg. Then, the electromotive force differences in the EAP sensor were determined.

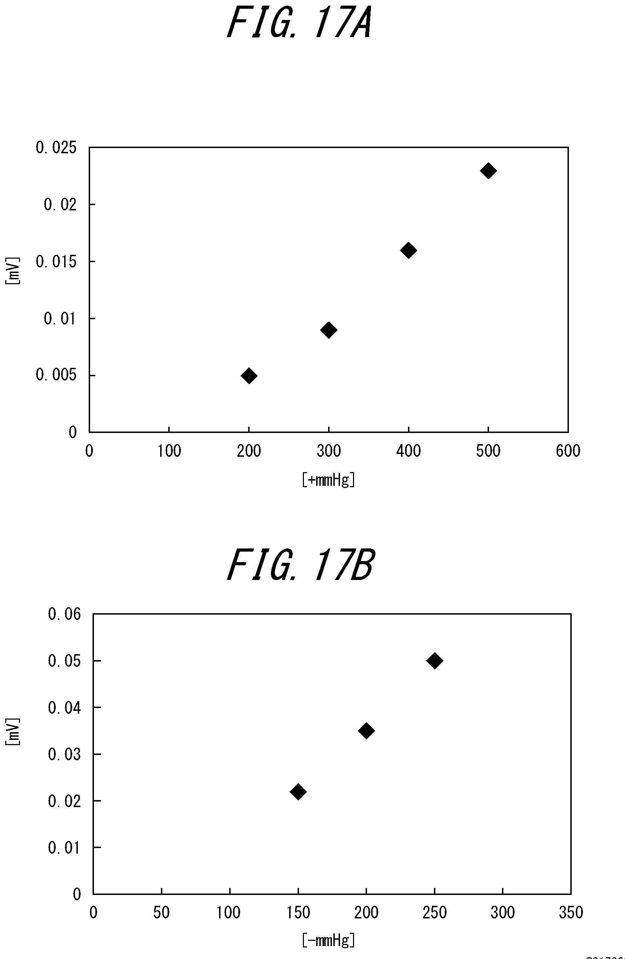

The averaged electromotive force differences under pressures inside the liquid feeding tube of +200, +300, +400, and +500 mmHg are listed in Table 2. Further, the relationship between the pressures inside the liquid feeding tube (+200, +300, +400, and +500 mmHg) and the electromotive force differences (averages) are depicted in FIG. 17A. In FIG. 17A, the horizontal axis indicates the pressures inside the liquid feeding tube (positive pressures) whereas the vertical axis indicates electromotive force differences under these pressures.

**Table 2**

| | | | | |
|---|---|---|---|---|
| Positive Pressures (mmHg) | 200 | 300 | 400 | 500 |
| Electromotive Force Differences (mV) | 0.005 | 0.009 | 0.016 | 0.023 |

As indicated in Table 2 and FIG. 17A, the electromotive force differences, i.e., differences between electromotive forces (induced voltages) of an EAP sensor and the initial voltage, increased with an increase in the positive pressures. The reason is considered that the surface of the liquid feeding tube deforms (expands) in a greater extent as a positive pressure increases. The more the surface of the liquid feeding tube deforms, the more the EAP sensor deforms and the higher the electromotive force difference becomes.

Next, the averaged electromotive force differences under pressures of -150, -200, and -250 mmHg in the liquid drawing tube are listed in Table 3. Further, the relationship between the pressures inside the liquid drawing tube (-150, -200, and -250 mmHg) and the electromotive force differences (averages) are depicted in FIG. 17B. In FIG. 17B, the horizontal axis indicates the pressures inside the liquid drawing tube (the absolute values of negative pressures) whereas the vertical axis indicates electromotive force differences under these pressures.

**Table 3**

| | | | |
|---|---|---|---|
| Negative Pressures (mmHg) | -150 | -200 | -250 |
| Electromotive Force Differences (mV) | 0.022 | 0.035 | 0.050 |

As indicated in Table 3 and FIG. 17B, the electromotive force differences, i.e., differences between electromotive forces (induced voltages) of an EAP sensor and the initial voltage, increased with an increase in the absolute values of negative pressures. The reason is considered that the surface of the liquid drawing tube deforms (contracts) in a greater extent as a negative pressure increases. The more the surface of the liquid drawing tube deforms, the more the EAP sensor deforms and the higher the electromotive force difference becomes.

Furthermore, FIGS. 17A and 17B indicate that the electromotive force differences had a proportional relationship with the absolute values of pressures (positive or negative pressures) in the circuit conduit. Specifically, it was found that the electromotive force differences were generally proportional to the absolute values of pressures in the circuit conduit. Here, an electromotive force difference is a difference between a voltage (electromotive force) induced in the EAP sensor in response to a deformation of a circuit conduit caused by a pressure inside the circuit conduit, and an induced voltage (electromotive force) in the EAP sensor in a predetermined condition (in the state where the pressure inside the circuit conduit is an initial pressure).

(Evaluations of Method of Attaching EAP Sensor using Attachment Jig 30)

Next, the method of attaching an EAP sensor using the attachment jig 30 described with reference to FIGS. 4-6B was evaluated. Initially, an attachment jig was fabricated. Specifically, a portion of a 20 mm-diameter resin tube was cut out in the longitudinal direction to fabricate an arc-shaped member (clamping section 31). Here, the arc-shaped member was fabricated such that the width of the opening (the gap between the ends of the arc) was 15 mm. A urethane film with a width of 20 mm and a thickness of 100 µm (manufactured by Takeda Sangyo Co., Ltd. under the product name of "Tough Grace") was then secured to one and the other ends of the arc-shaped member. Because the width of the urethane film was greater than the width of the opening of the arc-shaped member, the urethane film was secured so as to be accommodated within the arc. The properties of the urethane film are listed in Table 4.

**Table 4**

| Thickness | Direction | Modulus (Mpa) | | Breaking Strengths (Mpa) | Rupture Elongations (%) | Tensile Strengths (N) |
|---|---|---|---|---|---|---|
| | | 50% | 100% | | | |
| 100 µm | MD | 5.6 | 6.5 | 74.6 | 720.0 | 13.1 |
| | TD | 5.7 | 6.6 | 78.0 | 765.0 | 13.0 |

How the method of attaching EAP sensors using the fabricated attachment jig was evaluated will be described.

Initially, an EAP sensor was adhered to the urethane film of the attachment jig fabricated as above. The fabricated attachment jig was then pressed against a transparent tube made of vinyl chloride (with an outer diameter of 14.2 mm and an inner diameter of 9.5 mm) as a circuit conduit such that the EAP sensor adhered on the urethane film was made to contact the transparent tube. The transparent tube and the attachment jig were secured to each other with resin snappers (with sizes of 20 mm to 22 mm) manufactured by Kitaco Co., Ltd. EAP sensors sized to 10 mm × 50 mm and 5 mm × 30 mm, respectively, were used in the evaluations.

Next, one end of the transparent tube as a liquid drawing line 11A having the EAP sensor secured thereon by the attachment jig, was coupled to a casing containing tap water, and the other end was connected to a centrifugal pump (manufactured by Rouchon Industries Inc. under the product name of "MCP655-B"). In addition, one end of the transparent tube as a liquid feeding line 11B was coupled to the casing containing tap water, and the other end was connected to the centrifugal pump. Respective valves were provided to the transparent tubes as the liquid drawing line 11A and the liquid feeding line 11B.

In the measurement system described above, electromotive forces in the EAP sensors were measured three times by changing the pressures inside the transparent tubes by opening or closing the valves at the transparent tubes as the liquid drawing line 11A and the liquid feeding line 11B while water was being circulated by the centrifugal pump. Note that the fluid pressure at a pump speed of the centrifugal pump of 0 L/H was about 200 mgHg.

Table 5 lists the results of the three measurements of respective electromotive forces induced by deformations of the 10 mm × 50 mm EAP sensor, which were caused by pressures inside the transparent tube as the liquid drawing line 11A (negative pressures), and pressures inside the transparent tube as the liquid feeding line 11B (positive pressures).

**Table 5**

| | Electromotive force (mV) measured three times | | |
|---|---|---|---|
| Negative Pressure | 0.018 | 0.018 | 0.018 |
| Positive Pressure | 0.016 | 0.020 | 0.020 |

Table 5 indicates that stable measurement results were obtained with smaller errors both under the negative and positive pressures.

Table 6 lists the results of the three measurements of respective electromotive forces induced by deformations of the 5 mm × 30 mm EAP sensor, which were caused by pressures inside the transparent tube as the liquid drawing line 11A (negative pressures), and pressures inside the transparent tube as the liquid feeding line 11B (positive pressures).

**Table 6**

| | Electromotive force (mV) measured three times | | |
|---|---|---|---|
| Negative Pressure | 0.050 | 0.048 | 0.046 |
| Positive Pressure | 0.044 | 0.040 | 0.039 |

Table 6 indicates stable measurement results were also obtained with smaller errors both under the negative and positive pressures when the 5 mm × 30 mm EAP sensor was used. A comparison of the 5 mm × 30 mm EAP sensor with the 10 mm × 50 mm EAP sensor indicates that the 5 mm × 30 mm EAP sensor provided greater electromotive forces. Accordingly, it was found that greater electromotive forces can be achieved by optimizing the size of an EAP sensor.

While the present disclosure has been described with reference to the drawings and embodiments, it is noted that various modifications or variations may be easily made by those skilled in the art in the basis on the present disclosure. Accordingly, it is noted that such modifications or variations are encompassed within the scope of the present disclosure. For example, functions included in each block or the like may be rearranged unless such rearrangements are logically contradictory; or multiple blocks are combined into a single block or a single block may be divided.

### REFERENCE SIGNS LIST

- 1: liquid source
- 2: living human body
- 10: fluid system
- 11: circuit conduit
- 11A: liquid drawing line
- 11B: liquid feeding line
- 12: pump
- 13: EAP sensor
- 14: artificial lung
- 20: processor
- 30: attachment jig
- 31: clamping section
- 31a: one end
- 31b: the other end
- 32: pressing section
- 33: securing member
- 131: polymer element
- 132: polymer layer
- 133A, 133B: electrode layer
- 100: medical system

## Claims

1. A fluid system that allows a passage of a fluid through a circuit having a circuit conduit, the fluid system comprising:
a sensor disposed on an outer surface of the circuit conduit, the sensor comprising a polymer element comprising electrode layers provided at respective surfaces of an ion-conductive polymer layer,
wherein the sensor is configured to output a signal corresponding to a deformation of the circuit conduit.

2. The fluid system according to claim 1, wherein the sensor is secured to the outer surface of the circuit conduit by a protective film.

3. The fluid system according to claim 1 or 2, wherein the ion-conductive polymer layer is a polymer layer made of a fluororesin having a polar group.

4. The fluid system according to any one of claims 1-3, wherein the fluid system allows the passage of the fluid in the circuit using a pump,
the circuit conduit comprises a first line that allows a transportation of the fluid toward the pump and a second line that allows a transportation of the fluid away from the pump, and
the sensor is disposed on an outer surface of at least one of the first line and the second line.

5. The fluid system according to claim 4, wherein the polymer element has an approximate rectangular shape in plan view,
in a case where the sensor is disposed on an outer surface of the first line, the sensor is disposed such that long sides of the polymer element extend along a radial direction of the first line, and
in a case where the sensor is disposed on an outer surface of the second line, the sensor is disposed such that long sides of the polymer element extend along a longitudinal direction of the second line.

6. The fluid system according to any one of claims 1-5, wherein the fluid is blood.

7. The fluid system according to any one of claims 1-6, wherein a voltage corresponding to a deformation of the circuit conduit is induced in the sensor, and
an electromotive force difference has a proportional relationship with an absolute value of a pressure inside the circuit conduit, the electromotive force difference being a difference between the induced voltage in the sensor corresponding to the deformation of the circuit conduit caused by a pressure inside the circuit conduit, and an induced voltage in the sensor in a predetermined condition.

8. A medical system comprising:
the fluid system according to any one of claims 1-7; and
a processor configured to carry out a predetermined process according to the signal output from the sensor.

9. A sensor for use in the fluid system according to any one of claims 1-7, the sensor being configured to output the signal corresponding to the deformation of the circuit conduit.

10. A circuit conduit member comprising the sensor according to claim 9 disposed on an outer surface thereof.

11. A jig for attaching the sensor according to claim 9 to the circuit conduit, the jig comprising:
a clamping section having an arc shape in cross-sectional view; and
a pressing section accommodated within the arc of the clamping section,
wherein the sensor is to be placed on a surface of the pressing section opposite to a surface facing the clamping section, and
an accommodation of the circuit conduit within the arc of the clamping section causes the sensor to be pressed by the pressing section against an outer surface of the circuit conduit.

12. A method of attaching the sensor to the circuit conduit using the attachment jig according to claim 11, the method comprising:
placing the sensor on the surface of the pressing section opposite to the surface facing the clamping section;
accommodating the circuit conduit within the arc of the clamping section, thereby pressing the sensor by the pressing section against the outer surface of the circuit conduit; and
securing the clamping section and the circuit conduit to each other with a securing member.
